# EUROPEAN PATENT APPLICATION

(11) **EP 3 329 897 A1**
(43) Date of publication of application: **06.06.2018**
(21) Application number: 16829871.9
(22) Date of filing: 28.07.2016
(51) Int. Cl.: A61J 3/00, A61K 9/20, A61K 8/02

(54) **FREEZE-DRYING EXCIPIENT PREPARATION OF ARBITRARY SHAPE AND PREPARATION METHOD THEREFOR**

(30) Priority: 28.07.2015 CN 201510450377; 14.08.2015 CN 201510497752
(71) Applicant: Dong, Ling, Beijing 101312 (CN)
(72) Inventor: Dong, Ling, Beijing 101312 (CN)
(74) Representative: Tergau & Walkenhorst Patentanwälte PartGmbB
(86) International application number: PCT/CN2016/092074
(87) International publication number: WO 2017/016506

(57) **Abstract**

Disclosed are a freeze-drying excipient preparation of an arbitrary shape and a preparation method therefor, and in particular a method for preparing a freeze-drying excipient preparation of an arbitrary shape mainly containing an active ingredient and a binder by using multiple moulds, and a product prepared by the method.

## Description

### CROSS REFERENCE TO RELATED APPLICATIONS

This application claims the priority of Chinese Patent Application No. 201510450377.1, titled with "A SPHERICAL FREEZE-DRYING EXCIPIENT PREPARATION AND PREPARATION METHOD THEREFOR", filed with the Patent Office of the People's Republic of China on July 28, 2015, and the disclosures of which are hereby incorporated by reference.

This application also claims the priority of Chinese Patent Application No. 201510497752.8, titled with "METHOD OF PREPARING FREEZE-DRYING EXCIPIENT PREPARATION OF ARBITRARY SHAPE USING MULTIPLE MOULDS AND PRODUCT THEREOF", filed with the Patent Office of the People's Republic of China on August 14, 2015, and the disclosures of which are hereby incorporated by reference.

### FIELD

The present disclosure relates to a freeze-drying shaped preparation of an arbitrary shape and preparation method therefor, and in particular a preparation method for a freeze-drying shaped preparation of an arbitrary shape mainly containing an active ingredient and a binder by using multiple molds, and a product prepared by the method.

### BACKGROUND

Freeze-drying shaping technology is a shaping technology comprising adding skeleton support agent and binder to a flowable liquid, semi-solid or solid active ingredient (alternatively, the flowable liquid, semi-solid or solid active ingredient per se contains skeleton support agent and binder), and then filling the mixture into the molds for shaping by freeze-drying process. The preparation obtained by freeze-drying shaping technology is called freeze-drying shaped preparation.

This type of preparation is prepared by freeze-drying process, which can protect the thermosensitive ingredient from being damaged and have a fast disintegration and dissolution rate due to a great amount of micropores and channels produced by water sublimation. It has been widely used in a variety of fields such as oral disintegrating tablet, immediate release tablet, chewable tablet, and special cosmetic.

The freeze-drying shaped preparations on the market are mostly single form, and the molds used are traditional molds, that is, ordinary groove-type mold. Such traditional freeze-drying shaped preparations and preparation methods therefor have the following disadvantages:
(1) The shape of preparation is single due to the fixed shape of the molds, and cannot demold or can only demold from single direction; wherein "not demold" means shaping in a packing material having certain shape.
(2) Spherical, ellipsoid, irregular ball type and other special shapes are rare, because it is difficult to produce freeze-drying shaped preparation of special shape by using traditional preparation methods.
(3) The resulting tablet has an acute angle edge, and plurality of tablets having an acute angle edge after demolding are liable to wear after entering in the same package, resulting in inaccurate dosage of the drug.
(4) It is difficult to form a multi-layer structure due to the one-way filling, thus the structure of preparation is single.

### SUMMARY

In view of the above, the present disclosure provides a freeze-drying shaped preparation having an arbitrary shape and a method for producing the same. The present disclosure solves the problems that the shape of freeze-drying shaped preparation is single and is difficult to be prepared into spherical, ellipsoidal or irregular spherical, thus realize the manufacture of the same, resulting in more diverse forms of freeze-drying shaped preparations and controlling the production costs in a controllable range.

To achieve the above object of the present disclosure, the present disclosure provides the following technical solution:

The present disclosure provides a method for preparing a freeze-drying shaped preparation having an arbitrary shape using multiple molds, comprising the following steps:
A. preparing a mold by using a material having certain hardness and good thermal conductivity, wherein the mold is assembled from two or more parts, which is provided with a filling hole 13 and there is an assembly gap; and the assembly gap is not more than 10 mm, preferably not more than 5 mm;
B. precooling the mold at a precooling temperature of not more than 0°C;
C. preparing a solution containing water and a binder into a solution, emulsion or suspension to obtain a freeze-drying shaped preparation primary liquid;
D. filling the freeze-drying shaped preparation primary liquid into the mold through the filling hole 13 to completely or substantially occupy the space limited by the mold;
E. freezing and solidifying the freeze-drying shaped preparation primary liquid in the precooled mold; and
F. taking out the frozen and solidified freeze-drying shaped preparation primary liquid and freeze-drying the same to remove solvent and obtain a freeze-drying shaped preparation product.

The mold is assembled from two or more parts and there is no limit on the maximum number thereof. Preferably, the mold is assembled from 2 to 10 parts.

The material of the mold described in the preparation method should have certain hardness and a good thermal conductivity. The hardness of the material is represented by indentation hardness, and the indentation hardness thereof is between 0.1N and 100,000N, preferably not more than 90,000N, 80,000N, 70,000N, 60,000N, 50,000N, 40,000N, 30,000N or 20,000N. The thermal conductivity coefficient of the material should be not less than 0.01W/(m.k), where a material having a thermal conductivity coefficient of 0.05W/(m.k) to 1000W/(m.k) is preferred, and a material having a thermal conductivity of 0.2 W/(m.k) to 500W/(m.k) is the most preferred. The material may be one or more of metal, polymer material, ceramic, glass or the like. Also, the surface of the mold can be further subjected to surface treatment. The surface treatment of the mold can be coating, electroplating, acid-base washing, polishing, drawing, electrophoresis, PVD and other surface treatment methods, so that the freeze-drying shaped preparation primary liquid is well removed from the mold after freezing. The term "substantially occupy" in the above method means that it is 95% or more, preferably 98% or more, more preferably 99% or more, and most preferably 99.5% or more of the space limited by the mold.

The filling hole in the mold may be a filling connection opening with any shape, which is integrated with the upper mold or is independent of the presence of the upper and lower mold, and the shape thereof may be one of single-inlet and single-outlet, single-inlet and multi-outlet, multi-inlet and single-outlet, multi-inlet and multi-outlet, multi-layer inner and outer casings type filling connection opening, wherein the term "multi" means 2-100.

The obtained freeze-drying shaped preparation consists essentially of an active ingredient and a binder, in which the weight ratio of the binder to the active ingredient is 1:100 to 100:1.

The weight ratio of the binder to the active ingredient may be further preferably from 1:90 to 90:1, 1:80 to 80:1, 1:70 to 70:1, 1:60 to 60:1, 1:50 to 50:1, 1:40 to 40:1, 1:30 to 30:1, more preferably 1:20 to 20:1, 1:10 to 10:1, 1:9 to 9: 1, 1:8 to 8:1, 1:7 to 7:1, and most preferably 1:6 to 6:1, 1:5 to 5:1.

The freeze-drying shaped preparation provided by the present disclosure may further comprise other adjuvants such as backbone support agents, antioxidants, flavoring agents and flavors, transdermal or penetration adsorption enhancers, pH adjusting agents, and the like. The content of other adjuvant may be 0.1% to 5% of the obtained freeze-drying shaped preparation, preferably 0.1% to 3%.

The active ingredient used in the preparation method may be a substance which is water-soluble or water-insoluble, and the active ingredient may be one selected from a chemical pharmaceutical ingredient, an ingredient from traditional Chinese medicine, a natural extract, a bioactive ingredient, and a skin care beneficial ingredient, or a combination thereof.

The active ingredient is not particularly limited and may be selected from but not limited to a composition of one or more of the following ingredients.

### Chemical Pharmaceuticals (Pharmaceutical Active Ingredients):

Antipyretic, analgesic and anti-inflammatory drugs, such as aspirin, diflunisal, salsalate, acetaminophen, indomethacin, ibuprofen, naproxen, ketoprofen, pirprofen, suprofen, flurbiprofen, piroxicam, meloxicam, nimesulide, benzbromarone, etc.;
central stimulants, such as pemoline, adrafinil, piracetam, etc.;
drug for treating migraine, such as sumatriptan succinate;
analgesics, such as rotundine, buprenorphine, pentazocine, naloxone, etc.;
drugs for treating Parkinson's disease and senile dementia, such as levodopa, compound carbidopa, compound benserazide, amantadine hydrochloride, piribedil, profenamine, donepezil, huperzine-A, etc.;
antipsychataxia drugs, such as chlorpromazine, promethazine, pethidine, thioridazine, chlorprothixene, clozapine, sulpiride, tiapride, penfluridol, risperidone, etc.;
antiepileptic and anticonvulsant drugs, such as phenytoin sodium, carbamazepine, primidone, gabapentin, lamotrigine, sodium valproate, clonazepam, etc.;
sedative hypnotics, such as diazepam, nitrazepam, oxazepam, lorazepam, phenobarbital, etc.;
cholinesterase inhibitor, such as scopolamine, etc.;
antiarrhythmics, such as propiopyridine, tocainide, mexiletine, ethmozine, phenytoin sodium, propafenone, amiodarone, etc.;
anti-angina pectoris and anti-atherosclerosis drugs, such as propranolol, nifedipine, gemfibrozil, bezafibrate, lovastatin, simvastatin, pravastatin, etc.;
antihypertensive drugs, such as enalapril, captopril, hydrochlorothiazide, amlodipine, etc.;
adrenergic receptor blockers, shcu as acebutolol, alprenolol, etc.;
corticosteroids, such as betamethasone, cortisone acetate, etc.;
antidiabetic drugs, such as repaglinide, etc.;
anti-thyroid drugs, such as propylthiouracil, carbimazole, methimazole, etc.;
antihistamines, such as cetirizine hydrochloride, loratadine, etc.;
autacoids, such as dinoprostone, alprostadil, betahistine, etc .;
digestive system drugs, such as butylbromide scopolamine, granisetron hydrochloride, etc .;
blood system drugs, such as EPO, cobamamide, etc .;
urinary system drugs, such as azosemide, furosemide, etc .;
reproductive system drugs, such as estrogen, nandrolone phenylpropionate, etc.;
antiparasitic drugs, such as albendazole, cambendazole, etc.;
antineoplastic drugs, such as aminoglutethimide, amsacrine, etc.;
antimicrobial drugs, such as ampicillin, sulbenicillin sodium, etc.;
antibiotics such as amoxicillin, cephalexin, cefprozil, cefuroxime axetil, roxithromycin, erythromycin ethylsuccinate, josamycin and so on.

### Ingredients from Traditional Chinese Medicine:

active ingredient monomer from traditional Chinese medicine, such as breviscapine, artemisinin, huperzine-A, corydalis B, etc.;
single Chinese medicine extract and compound Chinese medicine extract, such as tanshinone extract, total salvianolic acid extract, compound Danshen dropping pill extract, compound Niuhuang shangqing pill extract, total saponin from ginseng stem and leaf, *Rhizoma Menispermi* extract, total saponin of ginseng, total saponin of American ginseng, breviscapine, *Sarcandrae Herba* extractum, total notoginsenoside, *Artemisia capillaris* extract, *Rhei Radix et Rhizoma* extractum, andrographolide, hawthorn leaf extract, total asiaticoside, ginkgo leaf extract, and so on.

### Natural Plant Extracts:

aloe extract, Chinese yam extract, cowberry extract, bitter gourd extract, echinacea extract, feverfew extract, mangosteen extract, pine needle and pine bark extract, acai berry extract, mulberry extract, elderberry extract, cranberry extract, astaxanthin, lycopene, green tea extract, grape seed and grape skin extract, glabridin, paeoniflorin, glycyrrhizic flavone, tree peony bark extract, and so on.

### Bioactive Ingredients:

EGF, bFGF, aFGF, KGF, IGF, NGF, TGF, HGH and the like.

### Skin Care Beneficial Ingredients:

vitamin A, vitamin B1, vitamin B2, vitamin B3, vitamin B6, vitamin B12, vitamin C, vitamin D, vitamin E, vitamin K, coenzyme, protease, metallothionein, pearl and its hydrolysate, milk and its extract, pollen and its extract, royal jelly, propolis, and so on.

The binder is an edible or pharmaceutically acceptable water-soluble polymeric material, which may be polysaccharide, polypeptide, protein, or synthetic polymer, or modified natural polymeric material or a mixture thereof. The binders include, but are not limited to, gelatin (gelatin, fish gelatin, bird gelatin, hydrolyzed gelatin, etc.), cellulose ether (methylcellulose, carboxymethylcellulose, hydroxypropylmethylcellulose, carboxyethylmethylcellulose, etc.), modified starch (pullulan, hydroxymethyl starch, etc.), hyaluronic acid, albumin, dextran, chitosan and its different molecular weight products, sodium alginate, PVP, PVA, polyethylene glycol, arabic gum, guar gum, xanthan gum, konjac gum, carrageenan, carbomer, agar, carrageenin, pectin or a combination thereof. The gum binder is collagen, gelatin, hydrolyzed gelatin, arabic gum, xanthan gum, carrageenan, pectin, konjac gum, carrageenin, locust bean gum, gum, locust bean gum; the cellulose ether binder is carboxymethylcellulose, carboxyethylcellulose, hydroxyethylmethylcellulose, hydroxypropylmethylcellulose or the like; the modified starch-derived binder is selected from pullulan, hydroxypropyl starch, hydroxypropylmethyl starch, pregelatinized starch, amylose, carboxymethyl starch, hydroxyethyl starch, hydroxypropyl starch, etc.; the polyamino acid is selected from polyglutamic acid, polyalanine, polylysine and the like; and the polysaccharide is selected from fucoidan, inulin and the like.

The freeze-drying shaped preparation may further comprise other adjuvant, which is one or more of backbone support agent, antioxidant, flavoring agent and essence, transdermal or penetration absorption enhancer, and pH adjusting agent.

The backbone support agent comprises but is not limited to sugar (such as maltose, trehalose, etc.), sugar alcohols (such as mannitol, sorbitol), amino acid having 2-12 carbon atoms (such as glycine, alanine, glutamic acid, etc.), as well as inorganic salt (such as sodium phosphate, aluminum silicate, etc.) and other substances.

The antioxidant includes but not limited to one or more of vitamin C and its derivatives, anthocyanin, resveratrol, and plant-derived polyphenol.

The flavoring agent and essence includes but not limited to one of a flavor with mint flavor, chocolate flavor, fruity flavor, vanilla flavor, coffee flavor, tea flavor, corn flavor, lemon flavor and milk flavor, or a mixture thereof.

The transdermal or penetration absorption enhancer includes but not limited to one of lecithin, saponin, sodium lauryl alcohol acid, azone, tween and span, or a mixture thereof.

The pH adjusting agent includes but not limited to one of citric acid, tartaric acid, sodium bicarbonate, carbonate, sodium carbonate and phosphate, or a mixture thereof.

The product prepared by the method of the present disclosure may be of any shape depending on the cavity enclosed by the mold. Preferably, the shape is tablet shape, capsule shape, soft capsule shape, spherical shape, ellipsoid shape or a shape of various characters, animals, plants, foods, graphic logos or cartoon characters. The product obtained by the method of the present disclosure may have no sharp corner and edge depending on the shape of the mold.

The present disclosure also provides a spherical freeze-drying shaped preparation prepared by the method, wherein the spherical freeze-drying shaped preparation is composed mainly of an active ingredient and a binder, and has a shape of spherical, ellipsoid, irregular ball and other special shapes without sharp corner and edge,

wherein the weight ratio of the binder to the active ingredient is 1:100 to 100:1; preferably 1:90 to 90:1, 1:80 to 80:1, 1:70 to 70:1, 1:60 to 60:1, 1:50 to 50:1, 1:40 to 40:1, 1:30 to 30:1; more preferably 1:20 to 20:1, 1:10 to 10:1, 1:9 to 9:1, 1:8 to 8:1, 1:7 to 7:1; most preferably 1:6 to 6:1, 1:5 to 5:1.

In some specific embodiments of the present disclosure, the mold described in the preparation method (1) is supported by a material having certain hardness and good thermal conductivity, including one or more of metal, polymeric material, ceramic and glass, or a mixture thereof.

In some specific embodiments of the present disclosure, the material of the mold may also be further subjected to a surface treatment to allow the freeze-drying shaped preparation liquid to be well removed from the mold after freezing.

In some specific embodiments of the present disclosure, the filling head for the freeze-drying shaped preparation in the preparation method (2) may be a pipetting device such as precise quantitative pipette, pipette, electronic pipette, or plunger pump, gear pump or peristaltic pump.

Another aspect of the present disclosure relates to a method for preparing the above mentioned spherical freeze-drying shaped preparation, which may be one of the following two preparation methods:
Preparation method (1):
   A. preparing a mold by using a material having a certain hardness and a good thermal conductivity, wherein the mold is assembled from multiple parts and assembly gap is not more than 10 mm;
   B. precooling the mold at a precooling temperature of not more than 0°C;
   C. preparing a solution or suspension of a material which includes but not limited to active ingredient, water, binder, backbone support agent and/or other adjuvant, and degassing the same to make a freeze-drying shaped preparation primary liquid;
   D. filling the freeze-drying shaped preparation primary liquid through a feeding port provided by the mold device, wherein the shear angle of the horizontal level therebetween the filling liquid and the mold directly contact with is an obtuse angle;
   E. freeze the mold and the freeze-drying shaped preparation primary liquid therein until the liquid is completely frozen and solidified;
   F. opening the mold device and taking out solidified freeze-drying shaped preparation primary liquid and freeze-drying the dame to remove solvent and obtain a freeze-drying shaped preparation product;
Preparation method (2):
   A. preparing a material which includes but not limited to active ingredient, water, binder, backbone support agent and/or other adjuvant into a solution or suspension and degassing the same to obtain a freeze-drying shaped preparation primary liquid;
   B. releasing a certain amount of freeze-drying shaped preparation primary liquid to an ultra-low temperature environment at a certain speed using a special filling head for the freeze-drying shaped preparation, to quickly solidify the freeze-drying shaped preparation primary liquid into spherical, elliptical, irregular ball or other special shapes; and
   C. freeze-drying the solidified freeze-drying shaped preparation to remove solvent and obtaining a freeze-drying shaped preparation product.

Another aspect of the present disclosure relates to a method for preparing the above mentioned spherical freeze-drying shaped preparation, which may be one of the following two preparation methods:
Preparation method (1):
   A. preparing a mold by using a material having a certain hardness and a good thermal conductivity, wherein the mold is assembled from multiple parts and assembly gap does not exceed 5 mm;
   B. precooling the mold at a precooling temperature of not more than -20°C;
   C. preparing a material which includes but not limited to active ingredient, water, binder, backbone support agent and/or other adjuvant into a solution or suspension and degassing the same to obtain a freeze-drying shaped preparation primary liquid;
   D. filling the freeze-drying shaped preparation primary liquid through a feeding port provided by the mold device, and the shear angle of the horizontal level therebetween the filling liquid and the mold directly contact with is an obtuse angle;
   E. freeze the mold and the freeze-drying shaped preparation primary liquid until the liquid is completely frozen and solidified; and
   F. opening the mold device and taking out the solidified freeze-drying shaped preparation primary liquid and freeze-drying the same to remove solvent and obtain a freeze-drying shaped preparation product;
Preparation method (2):
   A. preparing a material which includes but not limited to active ingredient, water, binder, backbone support agent and/or other adjuvant into a solution or suspension and degassing the same to obtain a freeze-drying shaped preparation primary liquid;
   B. releasing a certain amount of freeze-drying shaped preparation primary liquid to an ultra-low temperature environment at a certain speed using a special filling head for the freeze-drying shaped preparation, to quickly solidify the freeze-drying shaped preparation primary liquid into spherical, elliptical, irregular ball or other special shapes; and
   C. freeze-drying the solidified freeze-drying shaped preparation to remove solvent and obtaining a freeze-drying shaped preparation product.

In the above two methods, the weight ratio of the active ingredient and the binder to water is 1:100 to 100:1; further preferably 1:90 to 90:1, 1:80 to 80:1, 1:70 to 70:1, 1:60 to 60:1, 1:50 to 50:1, 1:40 to 40:1, 1:30 to 30:1; more preferably 1:20 to 20:1, 1:10 to 10:1, 1:9 to 9:1, 1:8 to 8:1, 1:7 to 7:1; most preferably 1:6 to 6:1, 1:5 to 5:1.

The method of degassing the primary liquid can be centrifugal degassing, vacuum degassing and ultrasonic degassing; the freezing may be carried out by means of liquid nitrogen or liquid, solid carbon dioxide spray cooling or circulating jacket cooling, turbo expander or cascade refrigeration, which can rapidly freeze solution, suspension or suspension to a solid at a temperature of from -20°C to -196°C.

The freeze-drying is carried out at a vacuum of 0.01 to 20mbar and a temperature ranging from -70°C to 50°C.

The mold in the preparation method (1) is made of a material having certain hardness and good thermal conductivity, which may be one or more of metal, polymeric material, ceramic, and glass. Also, the material of the mold may also be further subjected to a surface treatment to allow the freeze-drying shaped preparation primary liquid to be well removed from the mold after freezing.

The filling head in the preparation method (2) may be a pipetting device such as precise quantitative pipette, pipette, electronic pipette, or plunger pump, gear pump, or peristaltic pump.

The present disclosure provides a spherical freeze-drying shaped preparation having an arbitrary shape and a method for producing the same. The present disclosure solves the problems that the freeze-drying shaped preparation has a single shape and is difficult to be prepared into spherical, ellipsoidal or irregular spherical form, realizing the manufacture of the same. The present disclosure allows more diverse shapes of freeze-drying shaped preparation and controls the production costs in a controllable range.

### BRIEF DESCRIPTION OF DRAWINGS

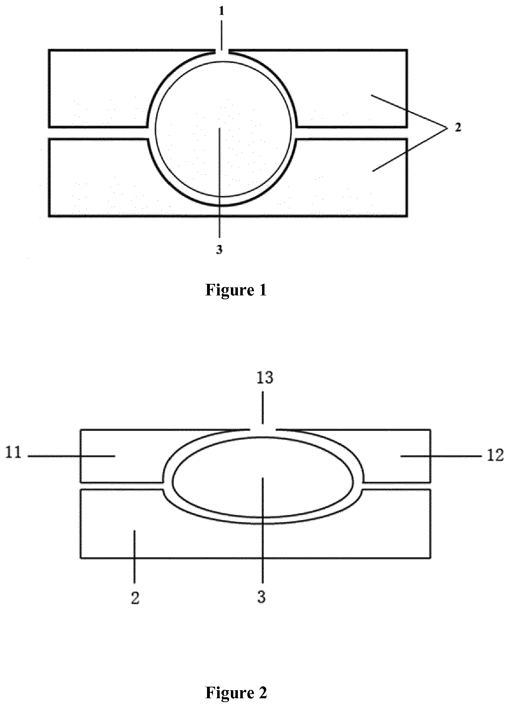

Figure 1 is the sectional view of a spherical freeze-drying shaped preparation in the mold, wherein: 1, the feeding port; 2, the assembly mold; 3, the frozen freeze-drying shaped preparation primary liquid.
Figure 2 is the sectional view of the mold part combination which can produce a product without sharp corner and edge; wherein: 11 and 12 together compose the upper mold; 13, filling hole; 2, lower mold; 3, the freeze-drying shaped preparation.

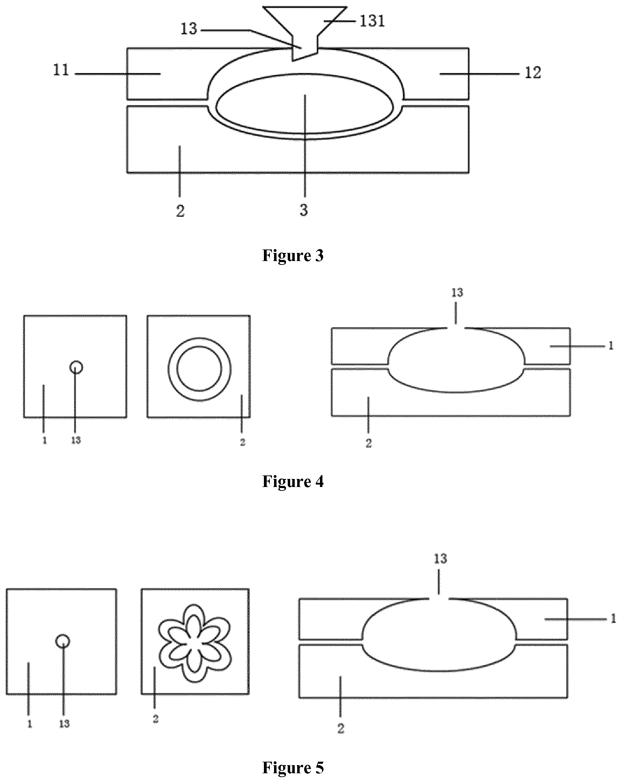

Figure 3 is the sectional view of the mold part combination which has an independent filling connection opening and can produce a product without sharp corner and edge; 131, independent, single-inlet and single-outlet type filling connection opening; 13, filling hole; 11 and 12 together compose the upper mold; 2, lower mold; 3, the freeze-drying shaped preparation.
Figure 4 is the view of the mold part combination of Example 3; wherein: 1, upper mold; 13, filling hole; 2, lower mold.
Figure 5 is the view of the mold part combination of Example 4; wherein: 1, upper mold; 13, filling hole; 2, the lower mold having a rose shape.

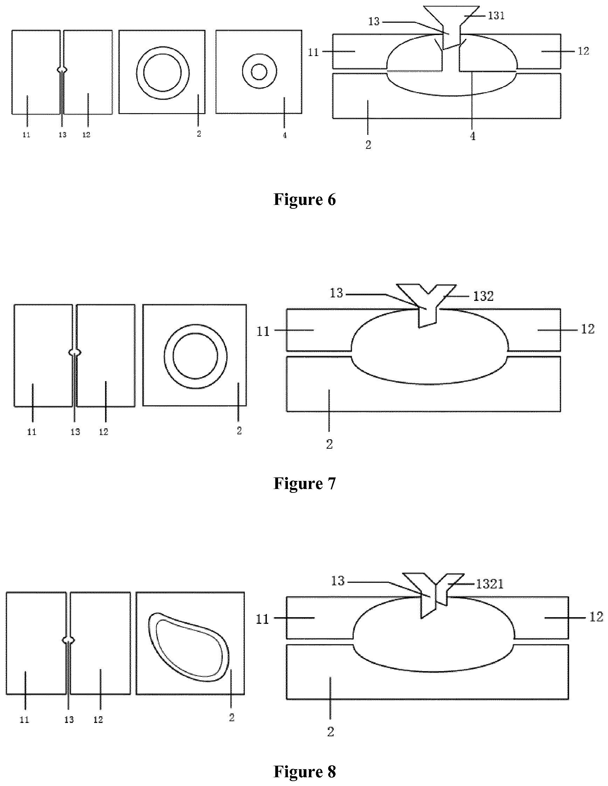

Figure 6 is the view of the mold part combination of Example 5; wherein: 131, independent, single-inlet and single-outlet type filling connection opening; 13, filling hole; 11 and 12 together compose the upper mold; 2, lower mold; 4, spacer.
Figure 7 is the view of the mold part combination of Example 6; wherein: 132, independent, double-inlet and single-outlet type filling connection opening; 13, filling hole; 11 and 12 together compose the upper mold; 2, lower mold.
Figure 8 is the view of the mold part combination of Example 7; wherein: 1321, independent, double-inlet and double-outlet type filling connection opening; 13, filling hole; 11 and 12 together compose the upper mold; 2, lower mold having a mango shape.

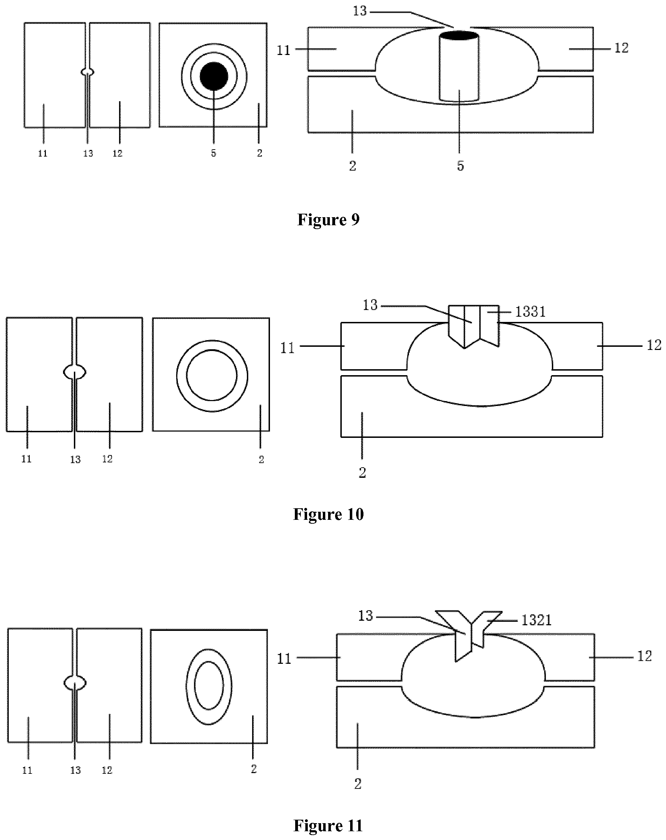

Figure 9 is the view of the mold part combination of Example 8; wherein: 11 and 12 together compose the upper mold; 13, filling hole; 2, lower mold; 5, central axis.
Figure 10 is the view of the mold part combination of Example 9; wherein: 1331, independent, triple-inlet and triple-outlet type filling connection opening; 13, filling hole; 11 and 12 together compose the upper mold; 2, lower mold.
Figure 11 is the view of the mold part combination of Example 10; wherein: 1321, independent, double-inlet and double-outlet type filling connection opening; 13, filling hole; 11 and 12 together compose the upper mold; 2, lower mold having an avocado shape.

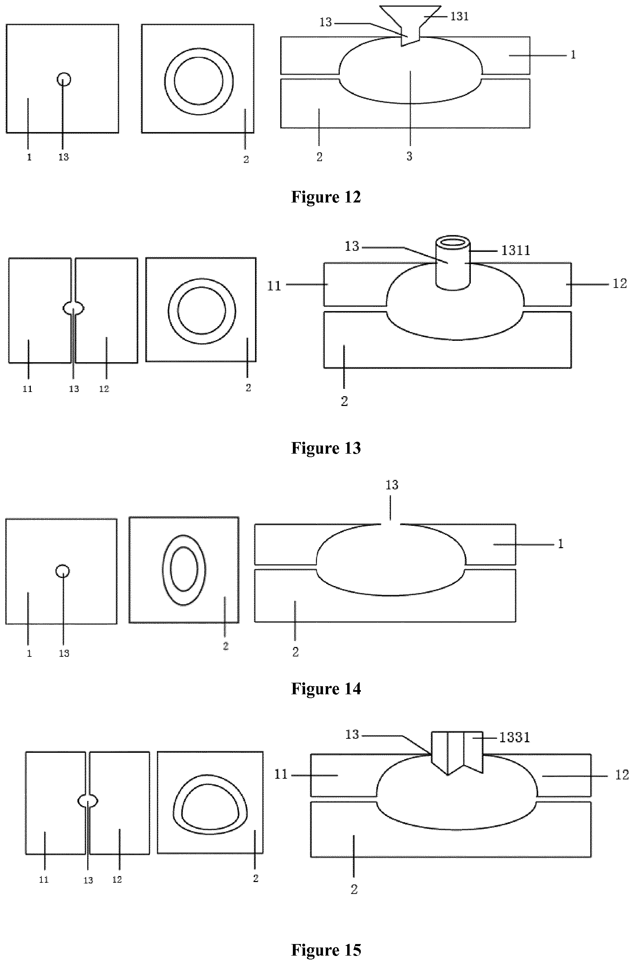

Figure 12 is the view of the mold part combination of Example 11; wherein: 1, upper mold; 13, filling hole; 131, independent, single-inlet and single-outlet type filling connection opening; 2, lower mold.
Figure 13 is the view of the mold part combination of Example 12; wherein: 1311, independent, casing type filling connection opening; 13, filling hole; 11 and 12 together compose the upper mold; 2, lower mold.
Figure 14 is the view of the mold part combination of Example 13; wherein: 1, upper mold; 13, filling hole; 2, elliptical lower mold.
Figure 15 is the view of the mold part combination of Example 14; wherein: 1331, independent, triple-inlet and triple-outlet type filling connection opening; 13, filling hole; 11 and 12 together compose the upper mold; 2, lower mold having a mabe pearl shape.

### DETAILED DESCRIPTION

The present disclosure discloses a spherical freeze-drying shaped preparation and preparation method thereof. Those skilled in the art can achieve it by modifying the process parameters appropriately in view of the contents of the present disclosure. It should be indicated particularly that all similar substitutions and modifications will be apparent to those skilled in the art and are considered to be in the scope of the present disclosure. The methods and applications of the present disclosure have been described by way of preferred embodiments, and it will be apparent to those skilled in the art that appropriate changes and combinations of the methods and applications described herein may be made without departing from the content, spirit and scope of the present disclosure, so as to realize and apply the technology of the present disclosure.

The materials and reagents used in the spherical freeze-drying shaped preparation and preparation method of the present disclosure are all commercially available in the market.

The present disclosure is further described with reference to the following examples:

### Example 1

(a) Spherical metal mold was precooled at -40°C;
(b) water was added to a mixture (cowberry extract : pullulan = 5:1) to prepare a solution, and the solution was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to be in spherical shape with a diameter of 5mm;
(e) the mold was open and the frozen spherical freeze-drying shaped preparation primary liquid was taken out and subjected to freeze-drying, and then was subjected to internal packing to give cowberry solid instant beverage.

### Example 2

(a) water was added to a mixture (rose extract : trehalose = 1:10) and stirred to prepare a solution, and the solution was subjected to centrifugal degassing to give a freeze-drying shaped preparation primary liquid;
(b) 0.1ml of the freeze-drying shaped preparation primary liquid was released to -80°C environment by hydraulic pump to freeze the freeze-drying shaped preparation primary liquid rapidly to be in an elliptical shape (length : width=2:1);
(c) the frozen freeze-drying shaped preparation primary liquid was subjected to freeze-drying and packed into penicillin bottle to which pure water was added before use to give a ready-to-use skin care product.

### Example 3

(a) A mold combination composed of two parts which can produce tablets without sharp corner and edge was taken and precooled to -40°C;
(b) water was added to a mixture (sodium ferulate : pullulan = 1:5) to prepare a solution, and the solution was subjected to centrifugal degassing to give a freeze-drying shaped preparation primary liquid;
(c) the freeze-drying shaped preparation primary liquid was slowly filled into the mold through filling hole 13 of the mold by using a pipette;
(d) the mold was open and the freeze-drying shaped preparation primary liquid was taken out after it was frozen and solidified, and was placed on a tray;
(e) the frozen freeze-drying shaped preparation primary liquid on the tray was subjected to freeze-drying to remove solvent to obtain freeze-drying shaped preparation; and
(f) the freeze-drying shaped preparation was subjected to internal packing to give sodium ferulate preparation.

### Example 4

(a) A mold combination composed of two parts which can produce rose shape tablet without sharp corner and edge was precooled to -10°C;
(b) A mixture of rose extract and trehalose in a ratio of 1:10 was stirred to prepare a solution, which was subjected to vacuum degassing to give a freeze-drying shaped preparation primary liquid;
(c) the freeze-drying shaped preparation primary liquid was slowly filled into the mold through filling hole 13 of the mold by using a charging pump;
(d) the freeze-drying shaped preparation primary liquid in the mold was subjected to freezing until the liquid was solidified completely;
(e) the mold was open and the frozen freeze-drying shaped preparation primary liquid was taken out and placed on a tray;
(f) the frozen freeze-drying shaped preparation primary liquid on the tray was subjected to freeze-drying to remove solvent to obtain a freeze-drying shaped preparation;
(g) the freeze-drying shaped preparation was subjected to internal packing, and pure water was added for dissolution before use to give a ready-to-use skin care product.

### Example 5

(a) A mold combination composed of three parts which can produce a two-layer tablet shape without sharp corner and edge was precooled to 0°C;
(b) component I (fresh pilose antler homogenate : ginseng extract = 2:1) and component II (fresh pilose antler homogenate : ginseng extract = 1:2) were prepared;
(c) water was added to component I and component II respectively, and stirred to prepare a solution and the solution was subjected to vacuum degassing to give freeze-drying shaped preparation primary liquid I and II, wherein the mass of component II was 0.1% of the total freeze-drying shaped preparation;
(d) the freeze-drying shaped preparation primary liquid I was slowly filled into the mold through filling hole 131 of the mold by using a pipette, then was frozen completely;
(e) spacer was removed; the freeze-drying shaped preparation primary liquid II was slowly filled into the mold through filling hole 131 of the mold by using a pipette until it was frozen into solid completely;
(f) the mold was open and the frozen freeze-drying shaped preparation primary liquid was taken out and placed on a tray;
(g) the frozen freeze-drying shaped preparation primary liquid on the tray was subjected to freeze-drying to remove solvent to obtain a freeze-drying shaped preparation; and
(h) the freeze-drying shaped preparation was subjected to internal packing to give ginseng-pilose antler buccal tablet.

### Example 6

(a) A mold combination composed of three parts (wherein the filling connection opening 132 is independent, double-inlet and single-outlet) which can produce two-layer tablet shape without sharp corner and edge was precooled to -10°C;
(b) component I (10% pullulan solution) and component II (PC : tween 80 = 5:1) were prepared; and the total mass of component II and component III was 0.1% of the total spherical freeze-drying shaped preparation;
(c) the solution I and solution II were stirred and subjected to centrifugal degassing to give freeze-drying shaped preparation primary liquid I and II, respectively;
(d) the freeze-drying shaped preparation primary liquid I and II were sequentially filled into the mold through the double-inlet and single-outlet filling connection opening by using a pipette until it was frozen completely;
(e) the mold was open and the frozen freeze-drying shaped preparation primary liquid was taken out and placed on a tray;
(f) the frozen freeze-drying shaped preparation primary liquid on the tray was subjected to freeze-drying to remove solvent to obtain a freeze-drying shaped preparation; and
(g) the freeze-drying shaped preparation was subjected to internal packing to give an oral buccal tablet containing PC.

### Example 7

(a) A mold combination composed of three parts (wherein the filling connection opening 1321 is independent, double-inlet and double-outlet) which can produce mango shape tablet without sharp corner and edge was precooled to -20°C;
(b) concentrated juice and pullulan (ratio of 10: 1) were stirred thoroughly and subjected to vacuum degassing to prepare component I; VC powder was component II; the mass of component II was 3% of the total freeze-drying shaped preparation;
(c) the freeze-drying shaped preparation component I and II were sequentially filled into the mold through the double-inlet and double-outlet filling connection opening 1321, respectively, by using a charging pump until it was frozen completely;
(d) the mold was open and the frozen freeze-drying shaped preparation primary liquid was taken out and placed on a tray;
(e) the frozen freeze-drying shaped preparation primary liquid on the tray was subjected to freeze-drying to remove solvent to obtain a freeze-drying shaped preparation; and
(f) the freeze-drying shaped preparation was subjected to internal packing to give a fruit candy tablet.

### Example 8

(a) A mold combination composed of four parts (wherein the filling connection opening 13 is not independent and the lower mold has a central axis) which can produce annulus tablet without sharp corner and edge was precooled to -40°C;
(b) a solution of sildenafil : pullulan : mannitol = 3: 1: 1 was stirred thoroughly and subjected to vacuum degassing to prepare a freeze-drying shaped preparation primary liquid;
(c) the freeze-drying shaped preparation primary liquid was filled into the mold through filling connection opening by using a charging pump;
(d) the mold was open and the frozen freeze-drying shaped preparation primary liquid was taken out and placed on a tray; the frozen freeze-drying shaped preparation primary liquid has a swim ring shape (outer diameter: 10mm, inner diameter: 8mm) since the lower mold has a central axis;
(e) the frozen freeze-drying shaped preparation primary liquid on the tray was subjected to freeze-drying to remove solvent to obtain an annular freeze-drying shaped preparation; and
(f) the freeze-drying shaped preparation was subjected to internal packing to form a final pharmaceutical product.

### Example 9

(a) A mold combination composed of three parts (wherein the filling connection opening 1331 is independent, triple-inlet and triple -outlet) which can produce spherical tablet without sharp corner and edge was precooled to -20°C;
(b) a solution of GTCC : vitamin D : lecithin = 5:1:1 was stirred thoroughly and subjected to vacuum degassing to prepare component I; water was added to hydrolyzed gelatin and pullulan (ratio of 1:1) and stirred thoroughly and subjected to vacuum degassing to prepare component II; water was added to concentrated juice and flavor (ratio of 10:1) and stirred thoroughly and subjected to vacuum degassing to prepare component III;
(c) component I, II and III were filled into the mold through the filling connection opening in the order of III, II, I by using a charging pump to form a structure of outer layer - component III, middle layer - component II and central layer - component I, which was frozen thoroughly; the total mass of component I and component III was 1% of the total freeze-drying shaped preparation;
(d) the mold was open and the frozen freeze-drying shaped preparation primary liquid was taken out and placed on a tray;
(e) the frozen freeze-drying shaped preparation primary liquid on the tray was subjected to freeze-drying to remove solvent to obtain a spherical freeze-drying shaped preparation; and
(f) the freeze-drying shaped preparation was subjected to internal packing to form a sandwich ball type nutritional supplement with an outer diameter of 30mm, middle diameter of 20mm and central diameter of 10mm.

### Example 10

(a) A mold combination composed of three parts (wherein the filling connection opening 1321 is independent, double-inlet and double-outlet) which can produce avocado shape three-dimensional tablet without sharp corner and edge was precooled to -30°C;
(b) shea butter and tween 80 (ratio of 3:1) were stirred thoroughly and subjected to vacuum degassing to prepare component I; water was added to a mixture of glutathione and pullulan (ratio of 5:1) and stirred thoroughly and subjected to vacuum degassing to prepare component II; laurocapram and modified lecithin (ratio of 1:3) were stirred thoroughly and subjected to vacuum degassing to prepare component III;
(c) component I, II and III were filled into the mold through the filling connection opening in the order of III, II, I by using a charging pump to form a structure of outer layer - component III, middle layer - component II and central layer - component I, which was frozen thoroughly; the total mass of component I and component III was 0.5% of the total freeze-drying shaped preparation;
(d) the mold was open and the frozen three-dimensional shape freeze-drying shaped preparation primary liquid was taken out and placed on a tray;
(e) the frozen freeze-drying shaped preparation primary liquid on the tray was subjected to freeze-drying to remove solvent to obtain a spherical freeze-drying shaped preparation with a diameter of 10mm; and
(f) the freeze-drying shaped preparation was subjected to internal packing to form a moisturizing skin care product in avocado shape.

### Example 11

(a) A mold combination composed of two parts (wherein the filling connection opening 131 is independent, single-inlet and single-outlet) which can produce a tablet in sheet shape without sharp corner and edge was precooled to -40°C;
(b) water was added to vitamin C and pullulan (ratio of 10:1) and stirred thoroughly and subjected to vacuum degassing to prepare a freeze-drying shaped preparation primary liquid;
(c) the freeze-drying shaped preparation primary liquid was filled into the mold through a filling connection opening by using a charging pump until it was frozen completely;
(d) the mold was open and the frozen three-dimensional shape freeze-drying shaped preparation primary liquid was taken out and placed on a tray;
(e) the frozen freeze-drying shaped preparation primary liquid on the tray was subjected to freeze-drying to remove solvent to obtain a freeze-drying shaped preparation; and
(f) the freeze-drying shaped preparation was subjected to internal packing to give vitamin C nutritional supplement.

### Example 12

(a) A mold combination composed of three parts (wherein the filling connection opening 1311 is independent, inside and outside casing type) which can produce spherical tablet without sharp corner and edge was precooled to -30°C;
(b) cocoa butter and lecithin (ratio of 3:1) were stirred thoroughly and subjected to vacuum degassing to prepare component I; water was added to a mixture of milk extract and pullulan (ratio of 5:1) were stirred thoroughly and subjected to vacuum degassing to component II; the mass of component I was 2.5% of the total freeze-drying shaped preparation;
(c) the freeze-drying shaped preparation primary liquid was filled into the mold through filling connection opening in the order of component II first and then component I through the outside casing tube of a casing type connection opening by using a charging pump, meanwhile inflation was carried out through the inside tube to make the freeze-drying shaped preparation primary liquid form a hollow structure when filing;
(d) the mold was open and the frozen freeze-drying shaped preparation primary liquid was taken out and placed on a tray;
(e) the frozen freeze-drying shaped preparation primary liquid on the tray was subjected to freeze-drying to remove solvent to give a freeze-drying shaped preparation; and
(f) the freeze-drying shaped preparation was subjected to internal packing to form a hollow sugar food.

### Example 13

(a) A mold combination composed of two molds (wherein the filling connection opening 13 is not independently assembled) which can produce elliptical tablet without sharp corner and edge was precooled to -40°C;
(b) a mixture of zolpidem tartrate : pullulan : mannitol (10:1:1) was stirred thoroughly and subjected to vacuum degassing to prepare a freeze-drying shaped preparation primary liquid;
(c) the freeze-drying shaped preparation primary liquid was filled into the mold through a filling connection opening by using a charging pump;
(d) the mold was open and the frozen freeze-drying shaped preparation primary liquid was taken out and placed on a tray;
(e) the frozen freeze-drying shaped preparation primary liquid on the tray was subjected to freeze-drying to remove solvent to give a freeze-drying shaped preparation; and
(f) the freeze-drying shaped preparation was subjected to internal packing to form a pharmaceutical product.

### Example 14

(a) A mold combination composed of three parts (wherein the filling connection opening 1331 is independent, triple-inlet and triple-outlet) which can produce a tablet in mabe pearl shape without sharp corner and edge was precooled to -50°C;
(b) water was added to a mixture of pearl hydrolyzate and pullulan (ratio of 10:1) and stirred thoroughly and subjected to vacuum degassing to prepare component I; nano-grade pearl powder was component II; GTCC and lecithin (ratio of 3:1) were stirred thoroughly and subjected to vacuum degassing to prepare component III;
(c) component I, II and III were filled into the mold through a filling connection opening in the order of I, II, III by using a charging pump to form a structure of outer layer - component I, middle layer - component II and central layer - component III, which was frozen thoroughly; the total mass of component II and component III was 2% of the total freeze-drying shaped preparation;
(d) the mold was open and the frozen three-dimensional shape freeze-drying shaped preparation primary liquid was taken out and placed on a tray;
(e) the frozen freeze-drying shaped preparation primary liquid on the tray was subjected to freeze-drying to remove solvent to obtain a freeze-drying shaped preparation in mabe pearl shape with a plane diameter of 4mm and a convex height of 3mm; and
(f) the freeze-drying shaped preparation was subjected to internal packing to form a skin care product.

### Example 15

(a) A spherical metal mold was precooled to -40°C;
(b) water was added to a mixture (dextran : hyaluronic acid = 1:100) to prepare a solution, which was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to spherical shape with a diameter of 15mm; and
(e) the mold was open and the frozen spherical freeze-drying shaped preparation primary liquid was taken out and subjected to freeze-drying, and then was subjected to internal packing to give a freeze-drying preparation.

### Example 16

(a) A spherical metal mold was precooled to -30°C;
(b) water was added to a mixture (ginseng extract : carbomer = 100:1) to prepare a solution, which was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to spherical shape with a diameter of 30mm; and
(e) the mold was open and the frozen spherical freeze-drying shaped preparation liquid was taken out and subjected to freeze-drying, and then was subjected to internal packing to give a freeze-drying preparation.

### Example 17

(a) A spherical metal mold was precooled to -50°C;
(b) water was added to a mixture (vitamin C: PVM/MA copolymer = 1:6) to prepare a solution, which was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to spherical shape with a diameter of 50mm; and
(e) the mold was open and the frozen spherical freeze-drying shaped preparation primary liquid was taken out and subjected to freeze-drying, and then was subjected to internal packing to give a freeze-drying preparation.

### Example 18

(a) A spherical metal mold was precooled to -70°C;
(b) water was added to a mixture (polyglutamic acid : mannitol = 6:1) to prepare a solution, which was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to spherical shape with a diameter of 100mm; and
(e) the mold was open and the frozen spherical freeze-drying shaped preparation primary liquid was taken out and subjected to freeze-drying, and then was subjected to internal packing to give a freeze-drying preparation.

### Example 19

(a) A spherical metal mold was precooled to -60°C;
(b) water was added to a mixture (milk protein : pullulan = 20:1) to prepare a solution, which was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to spherical shape with a diameter of 25mm; and
(e) the mold was open and the frozen spherical freeze-drying shaped preparation liquid was taken out and subjected to freeze-drying, and then was subjected to internal packing to give a freeze-drying preparation.

### Example 20

(a) A spherical metal mold was precooled to -80°C;
(b) water was added to a mixture (water-soluble astaxanthin : mannitol = 1:20) to prepare a solution, which was subjected to centrifugal degassing to give a freeze-drying shaped preparation primary liquid;
(c) the freeze-drying shaped preparation primary liquid was slowly filled into the mold through feeding port of the mold by using a pipette;
(d) the mold containing the freeze-drying shaped preparation primary liquid was subjected to quick freezing to solidify the freeze-drying shaped preparation primary liquid to spherical shape with a diameter of 18mm; and
(e) the mold was open and the frozen spherical freeze-drying shaped preparation liquid was taken out and subjected to freeze-drying, and then was subjected to internal packing to give a cowberry freeze-drying preparation.

### Example 21

By taking an example of manufacturing 1 million tablets, the production cost was compared between the production method provided by the present disclosure and the prior art method, and the results are shown in Table 1.

**Table 1. Comparison of Production Cost**

| Group | Prior art Method | | | Method provided by the present disclosure | | |
|---|---|---|---|---|---|---|
| | Mold Cost | Usage Amount | Total Mold Cost | Mold Cost | Usage Amount | Total Mold Cost |
| Example 1 | Conventional method cannot make a preparation. | | | 52 Yuan | 240 sets | 12480 Yuan |
| Example 2 | Conventional method cannot make a preparation. | | | 52 Yuan | 240 sets | 12480 Yuan |
| Example 3 | 100 Yuan | 600 sets | 60000 Yuan | 52 Yuan | 240 sets | 12480 Yuan |
| Example 4 | 100 Yuan | 600 sets | 60000 Yuan | 52 Yuan | 240 sets | 12480 Yuan |
| Example 5 | Conventional method cannot make a preparation. | | | 52 Yuan | 240 sets | 12480 Yuan |
| Example 6 | Conventional method cannot make a preparation. | | | 52 Yuan | 240 sets | 12480 Yuan |
| Example 7 | 100 Yuan | 600 sets | 60000 Yuan | 52 Yuan | 240 sets | 12480 Yuan |
| Example 8 | Conventional method cannot make a preparation. | | | 52 Yuan | 240 sets | 12480 Yuan |
| Example 9 | Conventional method cannot make a preparation. | | | 52 Yuan | 240 sets | 12480 Yuan |
| Example 10 | Conventional method cannot make a preparation. | | | 52 Yuan | 240 sets | 12480 Yuan |
| Example 11 | 100 Yuan | 600 sets | 60000 Yuan | 52 Yuan | 240 sets | 12480 Yuan |
| Example 12 | Conventional method cannot make a preparation. | | | 52 Yuan | 240 sets | 12480 Yuan |
| Example 13 | Conventional method cannot make a preparation. | | | 52 Yuan | 240 sets | 12480 Yuan |
| Example 14 | Conventional method cannot make a preparation. | | | 52 Yuan | 240 sets | 12480 Yuan |
| Example 15 | Conventional method cannot make a preparation. | | | 52 Yuan | 240 sets | 12480 Yuan |
| Example 16 | Conventional method cannot make a preparation. | | | 52 Yuan | 240 sets | 12480 Yuan |
| Example 17 | Conventional method cannot make a preparation. | | | 52 Yuan | 240 sets | 12480 Yuan |
| Example 18 | Conventional method cannot make a preparation. | | | 52 Yuan | 240 sets | 12480 Yuan |
| Example 19 | Conventional method cannot make a preparation. | | | 52 Yuan | 240 sets | 12480 Yuan |
| Example 20 | Conventional method cannot make a preparation. | | | 52 Yuan | 240 sets | 12480 Yuan |

Compared to the prior art, the preparation method provided by the present disclosure extremely significantly reduces (P<0.01) the production cost and enriches the morphology of the freeze-drying preparation.

The spherical freeze-drying preparation and preparation method thereof provided by the present disclosure are described in detail in the above. The principles and embodiments of the present disclosure have been described with reference to specific examples, and the description of the above embodiments is merely for the purpose of understanding the method of the invention and its core idea. It should be noted that it will be apparent to those skilled in the art that various improvements and modifications may be made to the present disclosure without departing from the principles of the invention, which fall within the scope of the claims of the present disclosure.

## Claims

1. A method of preparing freeze-drying shaped preparation having an arbitrary shape by using multiple molds, comprising the following steps:
A. preparing a mold by using a material having certain hardness and good thermal conductivity, wherein the mold is assembled from two or more parts, which is provided with a filling hole 13 and there may be an assembly gap due to processing accuracy; and the assembly gap is not more than 10 mm, preferably not more than 5 mm;
B. precooling the mold at a precooling temperature of not more than 0°C;
C. preparing a solution containing water and a binder into a solution, emulsion or suspension to obtain a freeze-drying shaped preparation primary liquid;
D. filling the freeze-drying shaped preparation primary liquid into the mold through the filling hole 13 to completely or substantially occupy the space limited by the mold;
E. freezing and solidifying the freeze-drying shaped preparation primary liquid in the precooled mold; and
F. taking out the frozen and solidified freeze-drying shaped preparation primary liquid and freeze-drying the same to remove solvent and obtain a freeze-drying shaped preparation product.

2. The method according to claim 1, wherein an active ingredient is added to the solution containing water and a binder in step C, and the active ingredient includes but not limited to one of a chemical pharmaceutical ingredient, an ingredient from traditional Chinese medicine, a natural extract, a bioactive ingredient, an orally administered food supplement and a skin care component or a combination thereof, which is beneficial to human body and animal.

3. The method according to claims 1 or 2, wherein the solution containing water and a binder in step C can further comprise other adjuvant, and the adjuvant is selected from one or more of backbone support agent, antioxidant, flavoring agent and flavor, transdermal or penetration adsorption enhancer, and pH adjusting agent.

4. The method according to claim 3, wherein the backbone support agent includes but not limited to sugar (such as maltose, trehalose, etc.), sugar alcohol (such as mannitol, sorbitol), amino acid having 2-12 carbon atoms (such as glycine, alanine, glutamic acid, etc.), as well as inorganic salt (such as sodium chloride, sodium phosphate, aluminum silicate, etc.); the antioxidant is selected from one of vitamin C and its derivatives, anthocyanin, resveratrol, plant-derived polyphenol or a mixture thereof; the flavoring agent and essence is selected from one of a flavor with mint flavor, chocolate flavor, fruity flavor, vanilla flavor, coffee flavor, tea flavor, corn flavor, lemon flavor or milk flavor, or a mixture thereof; the transdermal or penetration absorption enhancer is selected from one of lecithin, saponin, sodium laurylate, azone, tween, span or a mixture thereof; the pH adjusting agent is selected from one of citric acid, tartaric acid, sodium bicarbonate, carbonate, sodium carbonate, phosphate or a mixture thereof.

5. The method according to any one of claims 1-4, wherein the step E comprises: freezing and solidifying the freeze-drying shaped preparation primary liquid in the precooled mold; if the primary liquid is not frozen and solidified completely, the mold containing the primary liquid can be subjected to further freezing until the liquid is frozen completely.

6. The method according to any one of claims 1-5, wherein the mold is made of a material having an indentation hardness not less than 0.1N and a thermal conductivity not less than 0.01W/(m.k), and the material is selected from one or more of metal, polymer material, ceramic and glass.

7. The method according to any one of claims 1-6, wherein the filling hole 13 of the mold is an opening integrated with an upper mold or a filling connection opening 131 with any shape which is independent of the presence of the upper and lower mold, and the shape of the filling connection opening 131 may be one type of single-inlet and single-outlet, single-inlet and multi-outlet, multi-inlet and single-outlet, multi-inlet and multi-outlet, multi-layer inner and outer casings filling opening, wherein the "multi" means 2-100.

8. The method according to any one of claims 1-7, wherein surface of the mold can be further subjected to surface treatment so that the freeze-drying shaped preparation primary liquid can be well removed from the mold after freezing.

9. The method according to any one of claims 1-8, wherein the binder is an edible or pharmaceutically acceptable water-soluble polymeric material or a mixture thereof, which may be polysaccharide, polypeptide, protein, or synthetic polymer, or modified natural polymeric material or a mixture thereof, or natural material containing water-soluble polymeric material.

10. The method according to any one of claims 1-9, wherein the binder includes, but not limited to, gelatins (gelatin, fish gelatin, bird gelatin, hydrolyzed gelatin, etc.), cellulose ethers (methylcellulose, carboxymethylcellulose, hydroxypropylmethylcellulose, carboxyethylmethylcellulose, etc.), modified starch (pullulan, hydroxymethyl starch, etc.), hyaluronic acids, albumin, dextran, chitosan and its different molecular weight products, sodium alginate, PVP, PVA, polyethylene glycol, Arabic gum, guar gum, xanthan gum, konjac gum, carrageenan, carbomer, agar, carrageenin, pectin or a combination thereof, and natural material containing water-soluble polymeric material.

11. A product prepared by the method according to any one of claims 1-10.

12. The product according to claim 11, wherein the product has an arbitrary shape.

13. The product according to claim 12, wherein the product has no sharp corner and edge.

14. The product according to claims 12 or 13, which has a tablet shape, capsule shape, soft capsule shape, spherical shape, elliptical shape or a shape of various characters, animals, plants, foods, graphic logos or cartoon characters.

15. A spherical freeze-drying shaped preparation according to claim 14, wherein the spherical freeze-drying shaped preparation is mainly composed of an active ingredient and a binder and has a shape of spherical, elliptical, irregular ball and other special shapes without sharp corner and edge.

16. The spherical freeze-drying shaped preparation according to claim 15, wherein the active ingredient is selected from one of a chemical pharmaceutical ingredient, an ingredient from traditional Chinese medicine, a natural extract, a bioactive ingredient, and a skin care ingredient or a combination thereof; and the weight ratio of the binder to the active ingredient is from 1:100 to 100:1.

17. The spherical freeze-drying shaped preparation according to claim 15, wherein the binder is an edible or pharmaceutically acceptable water-soluble polymeric material or a mixture thereof, which includes polysaccharide, polypeptide, protein, synthetic polymer, modified natural polymeric material or a mixture thereof; and
the weight ratio of the binder to the active ingredient is 1:90 to 90:1, 1:80 to 80:1, 1:70 to 70:1, 1:60 to 60:1, 1:50 to 50:1, 1:40 to 40:1, 1:30 to 30:1; more preferably 1:20 to 20:1, 1:10 to 10:1, 1:9 to 9:1, 1:8 to 8:1, 1:7 to 7:1; most preferably 1:6 to 6:1, 1:5 to 5:1.

18. The spherical freeze-drying shaped preparation according to claim 17, wherein the binder includes but not limited to one of gelatins, cellulose ethers, modified starch, hyaluronic acids, albumin, dextran, chitosan and its different molecular weight products, sodium alginate, PVP, PVA, polyethylene glycol, Arabic gum, guar gum, xanthan gum, konjac gum, carrageenan, carbomer, agar, carrageenin and pectin, or a combination thereof;
the gelatins include one of gelatin, fish gelatin, bird gelatin, and hydrolyzed gelatin or a mixture thereof;
the cellulose ethers include one or methylcellulose, carboxymethylcellulose, hydroxypropylmethylcellulose, and carboxyethylmethylcellulose, or a mixture thereof;
the modified starch includes one of pullulanose, hydroxymethyl starch or a mixture thereof.

19. The spherical freeze-drying shaped preparation according to claim 15, further comprises other adjuvant, and the adjuvant is one of backbone support agent, antioxidant, flavoring agent and flavor, transdermal or penetration adsorption enhancer, and pH adjusting agent, or a mixture thereof; and
the content of said other adjuvant in the spherical freeze-drying shaped preparation is 0.1-5%, preferably 0.1-3%.

20. The spherical freeze-drying shaped preparation according to claim 19, wherein the backbone support agent includes but not limited to sugar, sugar alcohol, amino acid having 2-12 carbon atoms and/or inorganic salt;
the sugar includes one of maltose, trehalose or a mixture thereof;
the sugar alcohol includes one of mannitol, sorbitol or a mixture thereof;
the amino acid having 2-12 carbon atoms includes one of glycine, alanine, glutamic acid or a mixture thereof;
the inorganic salt includes one of sodium phosphate, aluminum silicate or a mixture thereof;
the antioxidant is selected from one of vitamin C and its derivatives, anthocyanin, resveratrol, plant-derived polyphenol or a mixture thereof; the flavoring agent and essence is selected from one of a flavor with mint flavor, chocolate flavor, fruity flavor, vanilla flavor, coffee flavor, tea flavor, corn flavor, lemon flavor or milk flavor, or a mixture thereof; the transdermal or penetration absorption enhancer is selected from one of lecithin, saponin, sodium laurylate, azone, tween, span or a mixture thereof; the pH adjusting agent is selected from one of citric acid, tartaric acid, sodium bicarbonate, carbonate, sodium carbonate, phosphate or a mixture thereof.

21. The method according to claim 15, wherein the preparation method can be one of the following two preparation methods:
Preparation method (1):
A. preparing a mold by using a material having a certain hardness and a good thermal conductivity, wherein the mold is assembled from multiple parts and assembly gap is not more than 10 mm;
B. precooling the mold at a precooling temperature of not more than 0°C;
C. preparing a solution or suspension of material and degassing the same to make a freeze-drying shaped preparation primary liquid;
D. filling the freeze-drying shaped preparation primary liquid through a feeding port provided by the mold device, wherein the shear angle of the horizontal level therebetween the filling liquid and the mold directly contact with is an obtuse angle;
E. freeze the mold and the freeze-drying shaped preparation primary liquid therein until the liquid is completely frozen and solidified;
F. opening the mold device and taking out solidified freeze-drying shaped preparation primary liquid and freeze-drying the dame to remove solvent and obtain a freeze-drying shaped preparation product;
wherein the material includes but not limited to active ingredient, water, binder, backbone support agent and/or other adjuvant;
Preparation method (2):
A. preparing material into a solution or suspension and degassing the same to obtain a freeze-drying shaped preparation primary liquid;
B. releasing a certain amount of freeze-drying shaped preparation primary liquid to an ultra-low temperature environment at a certain speed using a special filling head for the freeze-drying shaped preparation, to quickly solidify the freeze-drying shaped preparation primary liquid into spherical, elliptical, irregular ball or other special shapes;
C. freeze-drying the solidified freeze-drying shaped preparation to remove solvent and obtaining a freeze-drying shaped preparation product;
wherein the material includes but not limited to active ingredient, water, binder, backbone support agent and/or other adjuvant.

22. The method according to claim 15, wherein the preparation method can be one of the following two preparation methods:
Preparation method (1):
A. preparing a mold by using a material having a certain hardness and a good thermal conductivity, wherein the mold is assembled from multiple parts and assembly gap does not exceed 5 mm;
B. precooling the mold at a precooling temperature of not more than -20°C;
C. preparing material into a solution or suspension and degassing the same to obtain a freeze-drying shaped preparation primary liquid;
D. filling the freeze-drying shaped preparation primary liquid through a feeding port provided by the mold device, and the shear angle of the horizontal level therebetween the filling liquid and the mold directly contact with is an obtuse angle;
E. freeze the mold and the freeze-drying shaped preparation primary liquid until the liquid is completely frozen and solidified;
F. opening the mold device and taking out the solidified freeze-drying shaped preparation primary liquid and freeze-drying the same to remove solvent and obtain a freeze-drying shaped preparation product;
wherein the material includes but not limited to active ingredient, water, binder, backbone support agent and/or other adjuvant;
Preparation method (2):
A. preparing material into a solution or suspension and degassing the same to obtain a freeze-drying shaped preparation primary liquid;
B. releasing a certain amount of freeze-drying shaped preparation primary liquid to an ultra-low temperature environment at a certain speed using a special filling head for the freeze-drying shaped preparation, to quickly solidify the freeze-drying shaped preparation primary liquid into spherical, elliptical, irregular ball or other special shapes;
C. freeze-drying the solidified freeze-drying shaped preparation to remove solvent and obtaining a freeze-drying shaped preparation product;
wherein the material includes but not limited to active ingredient, water, binder, backbone support agent and/or other adjuvant.

23. The method according to claims 21 or 22, wherein the mold in the preparation method (1) is supported by a material having a certain hardness and a good thermal conductivity which includes one or more of metal, polymer material, ceramic and glass.

24. The method according to claim 23, wherein the material of the mold can be further subjected to surface treatment so that the freeze-drying shaped preparation primary liquid is well removed from the mold after freezing.

25. The method according to claims 21 or 22, wherein the filling head for the freeze-drying shaped preparation in the preparation method (2) can be a pipetting device such as precise quantitative pipette, pipette, electronic pipette, or plunger pump, gear pump, peristaltic pump.
